# EUROPÄISCHE PATENTSCHRIFT

(11) **EP 2 861 731 B1**
(45) Veröffentlichungstag und Bekanntmachung des Hinweises auf die Patenterteilung: **09.08.2017**
(21) Anmeldenummer: 13730524.9
(22) Anmeldetag: 14.06.2013
(51) Int. Cl.: C12N 11/00

(54) **VERFAHREN ZUR AKTIVIERUNG EINER OBERFLÄCHE DURCH ERHÖHUNG DER HYDROPHILIE UND/ODER FÜR DIE ANBINDUNG VON ZIELSTRUKTUREN**
METHOD FOR ACTIVATING A SURFACE BY INCREASING THE HYDROPHILICITY AND/OR FOR THE BONDING OF TARGET STRUCTURES
PROCÉDÉ POUR L'ACTIVATION D'UNE SURFACE PAR AUGMENTATION DE L'HYDROPHILIE ET/OU POUR LA LIAISON DE STRUCTURES CIBLES

(30) Priorität: 15.06.2012 DE 102012210163; 15.06.2012 US 201261660220 P
(43) Veröffentlichungstag der Anmeldung: 22.04.2015
(73) Patentinhaber: FRAUNHOFER-GESELLSCHAFT zur Förderung der angewandten Forschung e.V., 80636 München (DE)
(72) Erfinder: SZARDENINGS, Michael, 04299 Leipzig (DE); RISCHKA, Klaus, 21255 Tostedt (DE); GRUNWALD, Ingo, 28865 Lilienthal (DE); EISENSCHMIDT, Annika, 69124 Heldenberg (DE)
(74) Vertreter: Eisenführ Speiser
(86) Internationale Anmeldenummer: PCT/EP2013/062453
(87) Internationale Veröffentlichungsnummer: WO 2013/186392

(56) Entgegenhaltungen:
- WO-A1-2008/012236
- ANDRIJ PICH ET AL: "Composite Magnetic Particles as Carriers for Laccase fromTrametes versicolor", MACROMOLECULAR BIOSCIENCE, Bd. 6, Nr. 4, 12. April 2006 (2006-04-12), Seiten 301-310, XP055082926, ISSN: 1616-5187, DOI: 10.1002/mabi.200500192
- GODDARD ET AL: "Polymer surface modification for the attachment of bioactive compounds", PROGRESS IN POLYMER SCIENCE, PERGAMON PRESS, OXFORD, GB, Bd. 32, Nr. 7, 1. Juli 2007 (2007-07-01), Seiten 698-725, XP022133245, ISSN: 0079-6700, DOI: 10.1016/J.PROGPOLYMSCI.2007.04.002
- Michael Szardenings: "Enzymatic Treatment Re placing Plasma: Biocompatible Polymer Surfaces", , 1. Januar 2013 (2013-01-01), XP055082866, Gefunden im Internet: URL:http://www.izi.fraunhofer.de/fileadmin /Dokumente_Download/Leistungskatalog_2011/ Biocompatible_polymer_surfaces.pdf [gefunden am 2013-10-07]

## Beschreibung

Die Erfindung betrifft ein Verfahren zur Aktivierung einer Oberfläche durch Erhöhung der Hydrophilie und/oder für die Anbindung von Zielstrukturen.

Sie betrifft ferner die Verwendung einer Oxidoreduktase zur Aktivierung einer Oberfläche durch Erhöhung der Hydrophilie und/oder für die genannten Anbindungen.

Viele polymere Werkstoffe zeichnen sich durch eine hydrophobe Oberfläche aus. Um diese Materialien beschichten, lackieren oder verkleben zu können, ist eine Oberflächenaktivierung/ -modifizierung dieser Ausgangsstoffe unerlässlich. Viele polymere Materialien besitzen z. B.: nicht die erforderlichen Oberflächeneigenschaften, die für eine langzeitstabile Verklebung mit Klebstoffen, die leicht flüchtige organische Verbindungen enthalten, oder für die Schaffung von Kompositmaterialien notwendig ist.

Weiterhin besteht der Bedarf bei mikrofluidischen Bauteilen gezielt Strukturen zu hydrophilisieren.

Generell sind Oberflächenmodifikationen in Bereichen von Interesse, in denen die Oberflächenenergie des Substrates zur besseren Benetzbarkeit mit einer flüssigen Substanz verringert werden muss. Beispiele hierfür sind polymere Vliese aus PE oder PP, von Interesse z. B. für Watte oder Textilien.

Neben der technischen Anwendung steht auch die Verwendung von Plastikmaterialien in biologisch-medizinischen Anwendungen im Fokus. Zellkulturschalen werden meistens aus Polystyrol oder polyaliphatischen transparenten Polymeren hergestellt, die eine sehr niedrige Oberflächenenergie aufweisen, die sich nicht zur Ansiedlung von Zellen eignet.

Für die Arbeiten mit biologischen Systemen und/oder Molekülen besteht ein ständiger Bedarf an Materialien, die geeignet sind, eine lokale Fixierung der zu untersuchenden Strukturen zu gewährleisten. Im Falle von Zellen ist es in vielen Fällen nicht nur wünschenswert, sondern sogar notwendig, dass diese adhärent auf eine Oberfläche angesiedelt sind, um optimale Wachstums-/Proliferationsbedingungen zu gewährleisten.

Dabei stellt es sich für eine Vielzahl von Materialien, insbesondere auch Kunststoffe (polymere Materialien) als anspruchsvoll heraus, eine ausreichende Adhäsion der zu immobilisierenden Strukturen sicherzustellen. Ein Ansatz zur Aktivierung entsprechender Oberflächen ist z. B. Inkubieren mit scharfen Oxidationsmitteln wie z. B. Chromschwefelsaure. Dies setzt selbstverständlich voraus, dass eine sehr gründliche Entfernung der Oxidationsmittel erfolgt, zumindest für den Fall, dass empfindliche Moleküle/biologische Strukturen wie z. B. Zellen an den entsprechenden Oberflächen immobilisiert werden sollen.

Auch eine Anbindung der zu untersuchenden Strukturen, insbesondere von Zellen über Antikörper auf Oberflächen ist zwar zielstrukturspezifisch, häufig aber nicht ohne Einfluss auf die gebundene Struktur und zudem relativ kostenaufwendig.

Bis jetzt werden großtechnisch Oberflächen durch elektrische Vorbehandlung aktiviert. Zu den elektrischen Vorbehandlungsverfahren gehören die Corona- und die Niederdruckplasmabehandlung. Neben flüssigen Medien können auch Gase wie z. B. Ozon oder Fluor eingesetzt werden. Eine weitere Methode stellt die thermische Vorbehandlung der Oberfläche dar, wobei die Oberfläche kurzzeitig aufgeschmolzen und gleichzeitig chemisch verändert (oxidiert) wird. Als Wärmequelle dient hierbei eine Gasflamme oder ein Plasmabogen. Bei solchen Verfahren besteht das Risiko, dass auch toxische Verbindungen entstehen.

Die Verwendung von Enzym-basierten Verfahren wurde bisher in einigen Patentanmeldungen erwähnt (JP2003128835A, US2003017565A, US2010047533A, WO2011006041A1). Beispielsweise beschreibt die WO2008012236A1 (US2010047533A) ein Verfahren zur Erhöhung der Oberflächen-Hydrophilie eines Artikels aus Polyolefin oder Polyolefin-Copolymer, bei dem die Oberfläche mit einem aus Oxidoreduktasen ausgewählten Enzym behandelt wird (z. B. Cytochrom P450-Familie oder klassifizierte Enzyme EC 1,13 oder EC 1.14. Diese Verfahren unterscheiden sich jedoch von der unten beschriebenen Lösung.

Weiter sind im Stand der Technik verschiedene Beschichtungsvarianten bekannt, die das Anbinden von zu immobilisierenden Strukturen unterstützen sollen. Hier ist zumindest der zusätzliche Beschichtungsschritt zeit- und kostenaufwendig.

Vor diesem Hintergrund war es Aufgabe der vorliegenden Erfindung, ein Verfahren zur Aktivierung einer Oberfläche insbesondere für die Anbindung von Zielstrukturen, insbesondere Biomolekülen, wie Zellen und Proteinen zu geben, das unter milden Bedingungen, kostengünstig und zuverlässig in der Lage ist, entsprechende Oberflächen zu modifizieren.

Diese Aufgabe wird gelöst durch ein Verfahren zur Aktivierung einer Oberfläche durch Erhöhung der Hydrophilie und/oder für die Anbindung von Zielstrukturen, ausgewählt aus der Gruppe bestehend aus Proteinen, Zellen, Kohlenhydraten, Peptiden und Aminosäuren, umfassend den Schritt:
Die Inkubation der Oberfläche mit einer Oxidoreduktaseausgewählt ist aus der Gruppe E.C. 1.10 (Oxidoreduktasen, die an Diphenolen und ähnlichen Substanzen als Donoren agieren) nach der Klassifizierung nach dem IntEnz (Integrated relational Enzyme database des Swiss Institute of Bioinformatics (SIB)) und/oder der Gruppe der Oxidoreduktasen, die ohne Mediator, Cofaktor und Coenzym Substrate oxidieren können.

Unter Inkubation der Oberfläche mit einer Oxidoreduktase ist im Zusammenhang dieser Anmeldung zu verstehen, dass die Oberfläche mit der Oxidoreduktase in einer Form in Kontakt gebracht wird, in der diese üblicherweise ihre enzymatische Funktion ausüben kann. Dies bedeutet im Regelfall, dass die Oxidoreduktasen in Lösung und im Bereich der für das jeweilige Enzym individuell bestehenden optimalen Parameter (z.B. Arbeitstemperatur, pH-Wert) eingesetzt werden. Im pH-Bereich können im Sinne der Erfindung auch größere Abweichungen vom Optimalbereich (bezogen auf Kₘₐₓ) möglich sein.

Im Bereich der optimalen Arbeitstemperatur bedeutet dabei bevorzugt, dass die Oxidoreduktase bei einer Temperatur von maximal 5°C über einer optimalen Arbeitstemperatur und maximal 15°C unter der optimalen Arbeitstemperatur eingesetzt wird (jeweils bezogen auf Kₘₐₓ). Auch bei der Arbeitstemperatur können im Sinne der Erfindung größere Abweichungen vom Optimalbereich bezogen auf Kₘₐₓ möglich sein.

Unter Aktivierung einer Oberfläche für die Anbindung von Zielstrukturen ist in diesem Zusammenhang zu verstehen, dass gleiche Zielstrukturen nach Aktivierung gegenüber einer nicht-aktivierten Oberfläche vermehrt an die aktivierte Oberfläche anbinden.

Unter Erhöhung der Hydrophilie ist eine Verringerung des statischen Wasserrandwinkels im Sinne dieses Textes zu verstehen.

Überraschenderweise hat sich herausgestellt, dass allein die Inkubation mit entsprechenden Oxidoreduktasen insbesondere Laccasen sogar auch ohne Cofaktor, Mediator oder Coenzym bereits in der Lage ist, die Oberflächen insbesondere Polymeroberflächen an der Grenzfläche zu wässrigem Medium so zu aktivieren, dass eine Benetzbarkeit mit Wasser verbessert wird, d. h. dass die Hydrophilie steigt bzw. der Wasserrandwinkel sich verringert. Gleiches gilt für die Anbindung von Zielstrukturen, insbesondere Zellen und Proteinen zu aktivieren. Dies eröffnet eine Vielzahl von Vorteilen:
- Eine Inkubation mit Oxidoreduktasen, auf den entsprechenden Oberflächen lässt sich ohne großen apparativen Aufwand verwirklichen.
- Bei der Wahl geeigneter Oxidoreduktasen können die Bedingungen so gewählt werden, dass aufwendige Waschschritte nicht erforderlich sind.
- Ebenfalls bei der Auswahl geeigneter Oxidoreduktasen ist es möglich, die Zielstrukturen schon während des Aktivierungsschrittes in Kontakt mit der Aktivierungslösung kommen zu lassen, da die entsprechenden Enzyme die Funktionalität der Zielstruktur nicht oder wenigstens nicht wesentlich beeinflussen.
- Es stehen eine Vielzahl von Oxidoreduktasen zur Verfügung, so dass je nach Anwendungszweck beispielsweise auch menschliche Enzyme eingesetzt werden könnten.
- Es lassen sich durch das erfindungsgemäße Verfahren kovalente Anbindungen der Zielstruktur an die Oberflächen gewährleisten, wobei beispielweise im Falle von Zellen keine negativen Nebenwirkungen durch die Anbindung festgestellt werden konnten.
- Die aktivierte Oberfläche ist nach Anbindung der Zellstrukturen leicht durch die Zugabe von proteinhaltigem Medium inaktivierbar.
- Alle einzusetzenden Reagenzien sind vergleichsweise gut lagerfähig.
- Die verwendeten Reagenzien besitzen ein geringes Gefährdungspotential.
- Es ist kostengünstig möglich, Materialien für die Zellkultur bereitzustellen, bei denen das Grundmaterial (ohne Aktivierung) für den Verwendungszweck verbessert ist.
- Es ist möglich Oberflächen für eine Vielzahl von Anwendungen, wie z.B. Kleb-, Lackierungs- und Beschichtungsanwendungen zu aktivieren und deren Benetzungsverhalten zu verbessern.
- Es ist möglich, die Anwendbarkeit von mikrofluidischen Bauteilen durch erhöhte Hydrophilie zu verbessern.
- Durch das erfindungsgemäße Verfahren wird lediglich die oberste Schicht der Materialoberfläche modifiziert, so dass die wesentlichen Materialeigenschaften insbesondere die mechanischen Eigenschaften auch bei sehr dünnen Materialien erhalten bleiben.

Also stellt das erfindungsgemäße Verfahren eine Möglichkeit zur Aktivierung von Oberflächen für die Anbindung von Zielstrukturen insbesondere von Zellen und Proteinen dar, welches relativ einfach, kostengünstig, schnell und gegebenenfalls *in situ* die Aktivierung von Oberflächen für die Anbindung von Zielstrukturen gewährleisten kann.

Besonders bevorzugt ist die Oxidoreduktase ausgewählt aus der Gruppe E.C. 1.10.3 (mit Sauerstoff als Akzeptor), ganz besonders bevorzugt eine Oxidoreduktase aus den Klassen E.C. 1.10.3.2 (Laccasen) und E.C. 1.14.18.1 (Monophenol Monooxigenase). (Die Klassifizierung erfolgte jeweils nach IntEnz, s.o.), auch wenn die hier beschriebenen enzymatischen Aktivitäten anderen Klassen zugeschrieben werden müssten.

Die bevorzugten und besonders bevorzugten Oxidoreduktasen eignen sich für die Erzielung der oben genannten Vorteile in besonderer Weise.

Bevorzugt ist ein erfindungsgemäßes Verfahren, wobei die Inkubation ohne Zugabe einer Verbindung erfolgt, die eine di- oder tri-Hydroxyphenylgruppe umfasst.

Nach dem Stand der Technik musste man bislang davon ausgehen, dass die Oxidoreduktasen, insbesondere aber Laccasen und/oder Tyrosinoxidasen hydroxysubstituierte Phenylgruppen benötigen, um einen entsprechenden Oxidationsschritt zu katalysieren. Insbesondere im Falle von di- oder tri-Hydroxyphenylgruppen entsteht durch die Oxidation ein (reaktives) Chinon, welches mit den Oberflächen reagieren kann und so neue Anknüpfungspunkte für die Zielstrukturen zur Verfügung stellt. Dass für das erfindungsgemäße Verfahren zur Aktivierung ein solches Substrat nicht erforderlich ist oder eventuell auch auf unbehandelten Oberflächen (z.B.: Polystyrol, COC, Polypropylen) vorhanden ist, ist ein besonders überraschender Aspekt der vorliegenden Erfindung.

In diesem Sinne ist es bevorzugt, dass den eingesetzten Oxidoreduktase für das erfindungsgemäße Verfahren keine enzymspezifischen Substrate beigegeben werden.

Enzymspezifische Substrate sind dabei im Rahmen dieser Erfindung solche Substrate, die in der Literatur als typischerweise von diesem Enzym umgesetzte Substrate bezeichnet werden. Zu den enzymspezifischen Substraten im Sinne dieser Anmeldung zählen selbstverständlich nicht die zu aktivierenden Oberflächen.

Sofern kein zusätzliches enzymspezifisches Substrat für die Aktivierung der Oberflächen notwendig ist, bietet sich der Vorteil, dass weniger Chemikalien eingesetzt werden müssen, die Herstellung der entsprechenden Reaktionslösungen einfacher (und damit weniger fehlerbehaftet) ist und ein besser beherrschbares Reaktionssystem für die Aktivierung besteht.

Erfindungsgemäß bevorzugt ist ein Verfahren, wobei die Inkubation ohne Zugabe eines Redoxmediators erfolgt. Redoxmediatoren (und Cofaktoren oder Coenzyme) sind Verbindungen, die eine Redoxreaktion unterstützen, indem sie als Redoxpartner für die Oxidoreduktase und/oder die zu aktivierende Oberfläche zur Verfügung stehen. Im Sinne dieser Anmeldung ist Sauerstoff (O₂) ausdrücklich nicht unter die Gruppe der zuzugebenden Redoxpartner zu zählen.

Entsprechend dem Vorgesagten ist es erfindungsgemäß bevorzugt, dass das erfindungsgemäße Verfahren unter dem Einfluss von Sauerstoff, bevorzugt unter Lufteinfluss durchgeführt wird. Einfluss von Sauerstoff bzw. von Luft bedeutet dabei, dass sich das jeweilige Gas in freiem Austausch mit der die Oxidoreduktase enthaltenen Reaktionslösung befindet.

Alternativ bevorzugt kann ein erfindungsgemäßes Verfahren sein, bei dem die Inkubation unter Zugabe einer Verbindung erfolgt, die eine di- oder tri-Hydroxyphenylgruppe umfasst.

In vielen Fällen kann die Inkubation unter Zugabe eines typischen Substrates, insbesondere eines typischen Substrates für eine Laccase oder Tyrosinoxidase, zu einer Verbesserung des Aktivierungsgrades einer Oberfläche führen.

Unter Zugabe eines "typischen Substrates", insbesondere eines Katechols ist im Rahmen dieses Textes gemeint, dass die entsprechende Verbindung während der Aktivierung der Oberfläche in irgendeiner Weise dem Enzym zur Verfügung steht. Dies kann beispielsweise auch bedeuten, dass die entsprechenden Substrate (oder deren Vorstufen) bereits vor Zugabe der aktivierenden Oxidoreduktase auf die entsprechende Oberfläche aufgetragen und/oder ggf. an diese kovalent gebunden wurden.

Ein Vorteil des Einsatzes von Katecholen (di- oder tri-Hydroxyphenylgruppen umfassende Moleküle) ist auch darin zu sehen, dass diese Verbindungen in der Natur verhältnismäßig selten vorkommen, so dass das zur Aktivierung eingesetzte Enzym keine oder wenn, dann nur wenig Nebenreaktionen katalysiert.

Erfindungsgemäß besonders bevorzugt ist ein Verfahren, wobei die Inkubation unter Zugabe einer Verbindung erfolgt, ausgewählt aus der Gruppe bestehend aus DOPA, Kaffeesäure und Dopamin.

Diese Substrate eignen sich ganz besonders für die Aktivierung, die durch Laccasen durchgeführt wird.

Bevorzugt ist ein erfindungsgemäßes Verfahren, wobei das Material der Oberfläche ausgewählt ist aus der Gruppe bestehend aus Kunststoffen, Keramik, Glas, Kohlenhydraten und magnetische Partikel sowie Polyacryl und dessen Derivaten.

Allgemein bevorzugt ist das Material aus dem die Oberfläche besteht, ein ggf. modifiziertes und/oder oxidierbares polymeres Material.

Es können aber auch nicht-polymere Materialien für bestimmte Anwendungszwecke zum Aktivieren des Oberflächenmaterials bevorzugt sein.

Erfindungsgemäß bevorzugt ist, dass das Material der zu aktivierenden Oberfläche ausgewählt ist aus der Gruppe bestehend aus Polyolefinen, Polystyrol, Polypropylen, Polyethylen, Polycarbonat, Polyacryl, Polymethacryl, Cyloolefine und Cycloolefincopolymere und deren Derivaten sowie deren Mischungen.

Die bevorzugten Oberflächen eignen sich besonders gut für die Aktivierung im Rahmen des erfindungsgemäßen Verfahrens. Dabei sind Polystyrol und COC, insbesondere TOPAS ganz besonders bevorzugt.

Bevorzugt ist ein erfindungsgemäßes Verfahren, wobei die Aktivierung der Oberfläche in Anwesenheit der Zielstruktur erfolgt.

So ist es möglich, eine *in situ*-Aktivierung vorzunehmen, so dass aufwendige Zugabeschritte bzw. Waschschritte nicht erforderlich sind.

Bevorzugte Inkubationszeiten sind 20 Sekunden bis 16 Stunden, weiter bevorzugt 30 Sekunden bis 12 Stunden und besonders bevorzugt 1 Minute bis 3 Stunden.

Unter bestimmten Umständen kann auch ein erfindungsgemäßes Verfahren bevorzugt sein, wobei die Inkubation der Oberflächen mit einer Oxidoreduktase-Lösung erfolgt und die Lösung bei Raumtemperatur bis zur vollständigen Verdunstung des Lösungsmittels stehen gelassen wird.

Mit diesem Verfahren lassen sich besonders gute Aktivierungsergebnisse erzielen. Dabei sei betont, dass die erfindungsgemäß einzusetzende Oxidoreduktase bei dem erfindungsgemäßen Aktivierungsverfahren nicht kovalent an die Oberfläche gebunden wird.

Bevorzugt ist dementsprechend ein erfindungsgemäßes Verfahren, wobei nach der Inkubation die Oxidoreduktase vollständig wieder von der Oberfläche entfernt wird. Bevorzugt geschieht dies im Rahmen eines oder mehrerer Schritte.

"Vollständig entfernen" im Sinne des Vorgesagten bedeutet dabei, dass keine Oxidoreduktase an der Oberfläche mehr nachweisbar ist.

Erfindungsgemäß bevorzugt ist die einzusetzende Oxidoreduktase wie gesagt eine Laccase, bevorzugt wie sie in vielen holzabbauenden insbesondere Lignin abbauenden Organismen, ganz besonders Trametes spec. vorkommt, ganz bevorzugt erhältlich als Laccase C (ASA Spezialchemie, Wolfenbüttel, Deutschland).

Bevorzugt ist, dass das erfindungsgemäße Verfahren so durchgeführt wird, dass der statische Wasserrandwinkel gegenüber einer unbehandelten Oberfläche um ≥ 10,5°, bevorzugt ≥ 15° verringert wird.

Die Messung des Wasserrandwinkels erfolgt wie in Beispiel 6 beschrieben.

Teil der Erfindung ist auch die Verwendung einer Oxidoreduktase, die ausgewählt ist aus der Gruppe E.C. 1.10 (Oxidoreduktasen, die an Diphenolen und ähnlichen Substanzen als Donoren agieren) durch Erhöhung der Hydrophilie und/oder zur Aktivierung einer Oberfläche für die Anbindung Zielstrukturen, ausgewählt aus der Gruppe bestehend aus Proteinen, Zellen Proteinen, Zellen, Kohlenhydraten, Peptiden und Aminosäuren.

Wie bereits oben beschrieben ist insbesondere überraschend, dass Oxidoreduktasen sowohl mit als auch ohne spezifisches zu oxidierendes Substrat (Mediator/Cofaktor/Coenzym) in der Lage sind, Oberflächen zu aktivieren.

Teil der Offenbarung ist auch eine für die Anbindung von Zielstrukturen, ausgewählt aus der Gruppe bestehend aus Proteinen, Zellen, Kohlenhydraten, Peptiden und Aminosäuren an eine aktivierte Oberfläche, herstellbar oder hergestellt nach einem erfindungsgemäßen Verfahren.

Die durch das erfindungsgemäße Verfahren hergestellten Oberflächen bzw. Gegenstände, die eine solche Oberfläche tragen, weisen verbesserte Anbindungseigenschaften für die genannten Zielstrukturen auf. Sie sind mit dem erfindungsgemäßen Verfahren leicht und unter kostengünstigen sowie ohne großen zeit- und apparativen Aufwand möglich.

### Beispiele

### Beispiel 1: Verbindungseigenschaften von Laccase behandelten Oberflächen

Zur Untersuchung der Oberflächenmodifizierungseigenschaften von Laccase auf Polystyroloberflächen wurde ein allgemeines Versuchsschema verwendet (vgl. Tabelle 1). Die Versuche wurden bei Raumtemperatur (20-25°C) durchgeführt.

### Versuchsansätze:

A "ein-Topf": Laccase, das Substrat und BSA wurden gleichzeitig auf die Oberfläche gegeben, für eine Stunde inkubiert und der Überstand wurde auf die danach verbleibenden BSA-Konzentrationen untersucht.
B "Aktivierung": Laccasen und das Substrat wurden in das Well/die Petrischale für 10 Minuten gegeben, nach 10 Minuten Inkubationszeit wurde die Oberfläche mit Puffer gewaschen und BSA wurde hinzugefügt.
C "Inhibition": Wie B, vor der Zugabe von BSA wurde Ethanolamin hinzugefügt, um die Verbindungsstellen an der Oberfläche zu blockieren. Einwirkzeit: 15 Min. (Petrischale) oder 30 Min. (Well). Danach wurde wie bei B gewaschen und BSA hinzugefügt.
D: "Referenz": Die Bindungskonzentration von BSA auf eine Oberfläche, die nicht mit Laccase behandelt wurde, wurde untersucht (Inkubationszeit 60 Min. unter Anwesenheit des entsprechenden Substrates).

**Tabelle 1**

| Zeit [min] | A ("Ein-Topf") | B ("Aktivierung") | C ("Inhibition") | 1h BSA | D ("Referenz) |
|---|---|---|---|---|---|
| 0 | Laccase, BSA, Substrat | Laccase, Substrat | Laccase, Substrat | BSA | Substrat |
| 10 | | Waschen mit Puffer (pH 7) | Waschen mit Puffer (pH 7) | | |
| | | Zugabe von BSA | Zugabe von Ethanolamin | | |
| 25 (40) | | | Waschen mit Puffer (pH 7) | | |
| | | | Zugabe von BSA | | |
| 60 | Messung (Bradford) | | | Messung (Bradford) | Waschen mit Puffer (pH 7) |
| | | | | | Zugabe von BSA |
| 70 | | Messung (Bradford) | | | |
| BS (100) | | | Messung (Bradford) | | |
| 120 | | | | | Messung (Bradford) |

**Tabelle 2**

| Zusammenfassung der relevanten Konzentrationen: | | | |
|---|---|---|---|
| **Verbindung** | **Lösungsmittel** | **Konzentration** | **Volumen (Platte/Well)** |
| BSA (Fluka) | Phosphatpuffer pH 7 | 1 mg/mL | 1,5 mL (0,1%TWEEN)/ 750 µL |
| Kaffeesäure (Carl Roth GmbH) | Phosphatpuffer pH 7 | 1,5 x 10⁻⁴ M | 1,5 mL (0,1%TWEEN)/ 750 µL |
| Dopamin (Sigma Aldrich) | Phosphatpuffer pH 7 | 1,5 x 10⁻⁴ M | 1,5 mL (0,1%TWEEN)/ 750 µL |
| L-Dopa (Sigma Aldrich) | Phosphatpuffer pH 7 10 mM | 1,5 x 10⁻⁴M | 1,5 mL (0,1%TWEEN)/ 750 µL |
| Ethanolamin (Sigma Aldrich) | Wasser | 0,1 M | 1,5 mL (0,1%TWEEN)/ 25 µL |
| Laccase (ASA Spezialchemie GmbH) | Phosphatpuffer pH 7 10 mM | 20 mg/mL | |

Die Proteinkonzentration wurde nach Bradford bestimmt, dazu wurden die Überstände nach der in der Tabelle 1 angegeben, Zeit gesammelt und 250 µl jeder Probe zu 1 ml Bradford Reagenz gegeben. Für die 1-Pott Experimente wurde das Verhältnis angepasst, hier wurden 500 µl zu 750 µl Bradford Reagenz gegeben. 50 µl der jeweils resultierenden Lösung wurden in 950 µl Puffer gelöst und dann die Konzentration in einer Halbmikroküvette bei 595 nm bestimmt.

### Untersucht wurden 960 mm Petrischalen (Greiner).

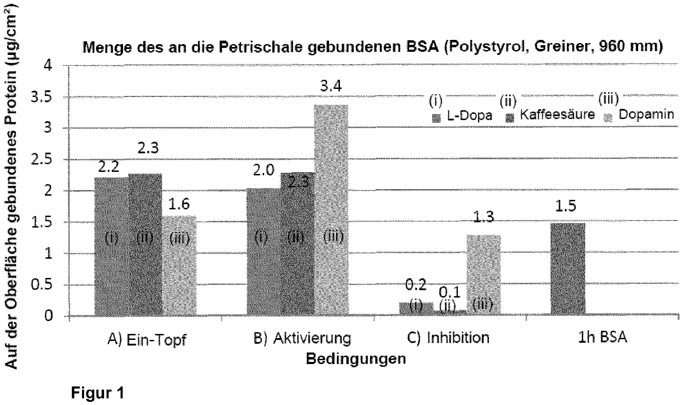

Fig. 1 stellt die Menge an BSA dar, die nach einer Stunde Inkubation gebunden wurde (ermittelt durch Differenzbildung zum Gesamtproteingehalt des Überstandes und nach der Inkubationszeit). Es zeigt sich, dass die BSA-Bindungskapazität der modifizierten Polystyroloberflächen in jedem Fall gesteigert wurde, teilweise bis über einen Faktor 2, verglichen mit der rechten Säule, die die Menge des BSA zeigt, das an die nicht-aktivierte Polystyroloberfläche bindet. Dabei zeigte sich die Aktivierung für alle drei untersuchten Substrate, nämlich Kaffeesäure, L-Dopa und Dopamin.

### Beispiel 2

In diesem Versuch sollte bestimmt werden, ob der in Beispiel 1 beobachtete Effekt auch für eine andere Polystyroloberfläche beobachtet werden kann. In diesem Fall wurden Falcom 240 mm 6 Wellplatten für Zellkulturen verwendet, das bedeutet, es handelt sich um eine Oberfläche, die bereits stahlenbehandelt war. Angewendet wurde das in Beispiel 1 beschriebene Versuchsschema.
Fig. 2 stellt die Ergebnisse einer Vorbehandlung mit dem jeweiligen Substrat ohne Laccase dar.
Fig. 3 stellt die Ergebnisse einer Vorbehandlung mit Laccase und L-Dopa dar.
Fig. 4 stellt die Ergebnisse einer Vorbehandlung mit nur Laccase dar.
Fig. 5 stellt die Ergebnisse einer Vorbehandlung mit Laccase und Kaffeesäure dar.
Fig. 6 stellt die Ergebnisse einer Vorbehandlung mit Laccase und Dopamin dar.

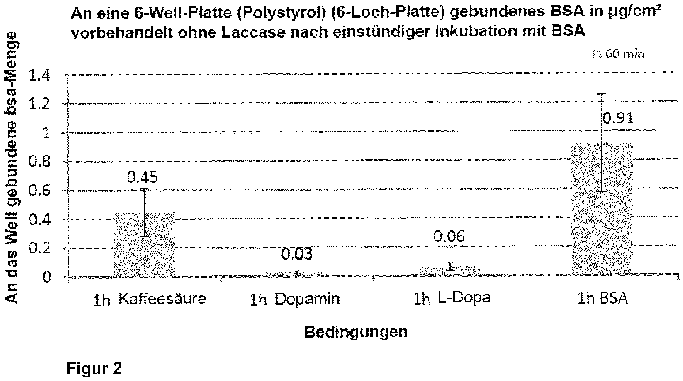

Aus Fig. 2 geht hervor, dass eine Vorbehandlung mit dem jeweiligen Substrat nicht zu einer Erhöhung der BSA-Bindungskapazität der behandelten Oberflächen führt, sofern die Behandlung nicht auch eine Behandlung mit Laccase beinhaltete.

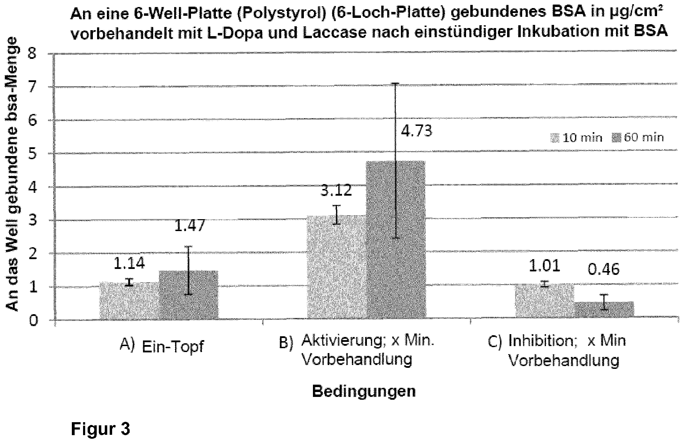

Fig. 3 zeigt u.a., dass eine Aktivierung mit Laccase in Kombination mit L-Dopa zu einer Aktivierung führt, wobei die Aktivierung mit zunehmender Vorbehandlungsdauer zunimmt und zu verbesserter Bindungskapazität führt.

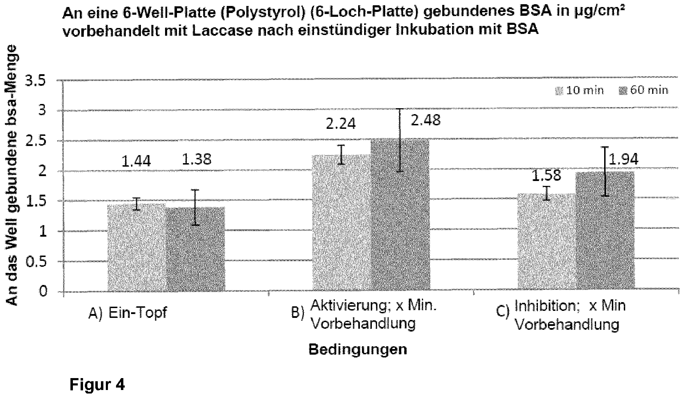

Fig. 4 zeigt, dass eine effektive Aktivierung auch möglich ist, sofern nur mit Laccase ohne weiteres Substrat vorbehandelt wird.

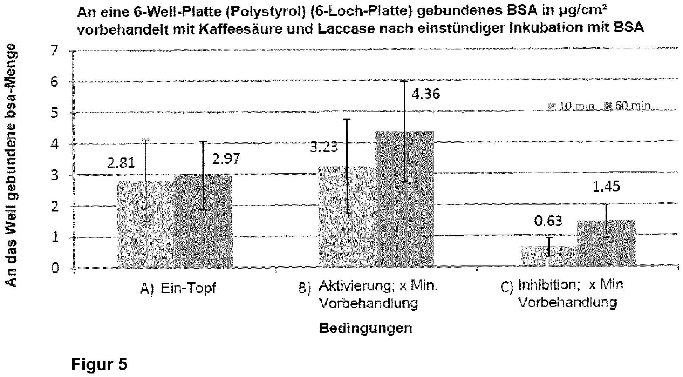

Fig. 5 zeigt die Effekte einer Vorbehandlung von Laccase mit Kaffeesäure, wobei jeweils analog zu den vorbeschriebenen Figuren eine Bindungskapazitätssteigerung zu beobachten war. Allerdings bemerkenswert in diesem Zusammenhang ist, dass Kaffeesäure keine Aminogruppe umfasst, so dass man davon ausgehen kann, dass der eigentliche Aktivierungsmechanismus unabhängig von dieser Gruppe ist.

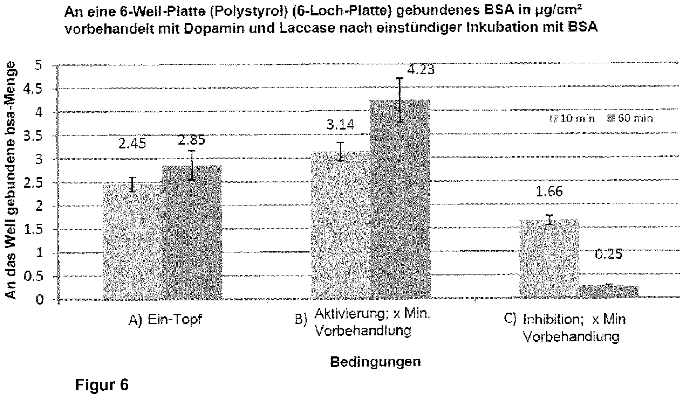

Fig. 6 bildet die entsprechenden Ergebnisse mit Dopamin als alternativem Substrat für die Laccase in der Vorbehandlung ab. Auch hier ist eine analoge Bindungskapazitätssteigerung zu beobachten.

### Beispiel 3: Bindungsversuche mit Zellen

Zellen der Zelllinie HaCaT P14 wurden auf analog dem Beispiel 2 vorbehandelte 160 mm Polystyrol Zellsuspensionskulturplatten gegeben.

Auch hier ließ sich eine deutliche Adhäsionskapazitätssteigerung der Platten analog zu den Beispielen 1 und 2 zeigen. Noch weiter verbesserte Ergebnisse ließen sich erzielen, indem die jeweilige Vorbehandlungslösung (Inkubationslösung) über Nacht eintrocknen gelassen wurde und anschließend die Zelladhäsionsversuche durchgeführt wurden.

### Beispiel 4: Aktivierung von Petrischalen aus TOPAS

Petrischalen aus TOPAS (COC-Polymer) der Firma ibidi, Martinsried (µ-Dish) wurde wie folgt verwendet:
1. ibidi µ-Dish, unbehandelt: Normale hydrophobe Gefäße
2. ibidi µ-Dish, ibidi treat: Kommerziell erhältlich mit plasmabehandelter Oberfläche
3. ibidi µ-Dish, erfindungsgemäß behandelt mit 0,5 U Laccase in 2 ml in 10 mmol Phosphatpulver, pH 7,0 (Einwirkzeit 20 Minuten)
4. ibidi µ-Dish, erfindungsgemäß behandelt mit 0,1 U Laccase in 2 ml in 10 mmol Phopshatpulver, pH 7 (Einwirkzeit 60 Minuten)

Nach der Einwirkzeit wurden die Platten mit deionisiertem Wasser gewaschen und mit Stickstoff getrocknet. Dabei ergab sich, dass die Proben 3 und 4 (erfindungsgemäß) verglichen mit der unbehandelten Probe (Probe 1) bei Auftragen eines Wassertropfens eine Spreizung des Tropfens zeigten, die deutlich höher und sogar vergleichbar mit der plasmabehandelten Probe (Probe 2) war. Bei der erfindungsgemäßen Probe 3 zeigten sich Veränderungen in der Oberflächenspannung bereits nach 5-10 Minuten, während bei der erfindungsgemäßen Probe 4 diese Änderungen zwischen 15 und 45 Minuten auftraten.

### Beispiel 5: Oberflächenuntersuchung

### mittelsRöntgenphotoelektronenspektroskopie (XPS)

### Röntgenphotoelektronenspektroskopie (XPS)

Messprinzip: Die Röntgenphotoelektronenspektroskopie (XPS) beruht auf dem äußeren Photoeffekt, bei dem durch Röntgenstrahlung Photoelektronen aus einer Festkörperoberfläche ausgelöst werden. Die Bestimmung der kinetischen Energie dieser Elektronen erlaubt Rückschlüsse auf die chemische Zusammensetzung und die elektronische Beschaffenheit der untersuchten Probenoberfläche. Die Analyse ist oberflächenempfindlich. Die zugehörige Informationstiefe, die durch die begrenzte Austrittstiefe der emittierten Elektronen bestimmt wird, beträgt ca. 10 nm. Die Methode erlaubt eine quantitative Bestimmung der chemischen Zusammensetzung des oberflächennahen Bereiches. Dabei werden alle Elemente mit Ausnahme von Wasserstoff und Helium erfasst. Die Nachweisempfindlichkeit der Methode ist elementspezifisch und liegt bei ca. 0.1 at%, d.h. ca. 1000 ppm. Zur Kompensation von Aufladungseffekten wird die C1s-Hauptphotolinie bei der Auswertung auf 285 eV festgelegt, dadurch verschieben sich die Lagen weiteren Photolinien entsprechend.

Messparameter: Die XPS-Untersuchungen erfolgten mit einem Thermo K-Alpha K1102-System mit vorgeschalteter Stickstoff-Glovebox für die Handhabung luftempfindlicher Proben. Parameter: Abnahmewinkel der Photoelektronen 0°, monochromatisierte AlKα-Anregung, Constant Analyser Energy-Mode (CAE) mit 150 eV Passenergie in Übersichtsspektren sowie 20 eV in energetisch hochaufgelösten Linienspektren, Analysenfläche: 0,40 mm. Die Neutralisation von elektrisch nichtleitenden Proben erfolgt durch eine Kombination von niederenergetischen Elektronen und niederenergetischen Argon-Ionen.

### XPS-Ergebnisse

Die quantitative Auswertung der XPS-Übersichtsspektren zu den untersuchten Proben ergibt die nachfolgenden tabellarisch aufgeführten elementaren chemischen Zusammensetzungen des oberflächennahen Bereiches der analysierten Stellen (vgl. Tabelle 1). Die Konzentrationen der detektierten Elemente, mit Ausnahme von Wasserstoff und Helium, sind in Atomprozent (at%) angegeben.

**Tabelle 1: XPS-Untersuchung an folgenden Proben, chemische Zusammensetzungen der Oberflächen in Atomprozent (at%):**

| | **C (at%)** | **O (at%)** | **N (at%)** | **Si (at%)** | **P (at%)** | **Ca (at%)** | **S (at%)** | **Na (at%)** | **F (at%)** | **Mg (at%)** |
|---|---|---|---|---|---|---|---|---|---|---|
| Platte 1: unbehan delte Platte mit Ac-Puffer | 96,2 | 3,1 | 0,1 | <0,1 | <0,1 | 0,1 | 0,3 | - | - | - |
| Platte 2: Plasmabehande Ite Platte mit Ac-Puffer | 83,0 | 13,6 | 0,7 | 1,3 | - | 0,4 | - | 0,8 | 0,2 | 0,1 |
| Platte 3: unbehan delte Platte + Lacase + Ac-Puffer | 83,3 | 10,4 | 5,0 | 0,4 | <0,1 | 0,1 | 0,1 | 0,2 | 0,4 | <0,1 |

Bei den Platten 1 und 2 handelt es sich um dasselbe Material wie die Platten 1 und 2 aus Beispiel 4. Bei Platte 3 handelt es sich um dasselbe Material wie Platte 1. Alle drei Platten wurden in je 4ml Acetat-Puffer (2 mol/l, pH=4,7) für 15 Stunden inkubiert, wobei Platte 3 (erfindungsgemäß) Inkubationslösung (4 U Laccase in 4 ml Acetat-Puffer) enthielt.

Im Ergebnis zeigt sich, dass es mit dem erfindungsgemäßen Verfahren möglich ist, eine nennenswerte Menge Sauerstoff an der Oberfläche einzubauen. Ferner zeigt sich, dass auch zusätzlicher Stickstoff eingebaut wird. Dementsprechend sind erfindungsgemäß hergestellte Oberflächen bevorzugt, die einen Stickstoffanteil von ≥ 3 % bezogen auf die Gesamtzahl der an der Oberfläche mittels XPS bestimmbaren Atome (ohne H) besitzt.

### Beispiel 6: Wasserrandwinkelbestimmung

Die Proben wurden wie folgt vorbereitet: Vor Beginn der Behandlung wurden die Proben mit Isopropanol abgewischt und man ließ die gereinigten Substrate für 15 Minuten im Abzug zur Entfernung von verbleibenden Isopropanol-Spuren liegen. Anschließend wurden die Substrate für 3 h in je 50 ml einer Lösung aus a) Laccase in 0,1M-Phosphatpuffer (0,5 U / ml), b) denaturierter Laccase in 0,1M-Phosphatpuffer (ausgehende Konzentration vor Denaturierung 0,5 U / ml) und c) demineralisiertes Wasser gelegt. Nach beendeter Auslagerung wurden die jeweiligen Substrate in je 50 ml demineralisiertes Wasser gewaschen und im Stickstoff-Strom getrocknet.

Nachfolgend wurde der statische Kontaktwinkel mit Wasser gemäß der DIN 55660-2 (Dezember 2011) bestimmt.

Die Ergebnisse sind in Tabelle 2 zusammengefasst:

| Probe | | Kontaktwinkel [°] | | |
|---|---|---|---|---|
| PS | -H | 91,4 | 91,0 | 93,5 |
| | -L | 72,2 | 72,6 | 68,2 |
| | -D | 81,2 | 84,9 | 84,3 |
| PE | -H | 94,0 | 95,3 | 92,2 |
| | -L | 83,1 | 83,4 | 81,3 |
| | -D | 93,8 | 88,6 | 92,0 |
| PC | -H | 90,4 | 90,4 | 89,0 |
| | -L | 61,3 | 60,2 | 59,9 |
| | -D | 82,1 | 82,3 | 86,6 |

| | | | | |
|---|---|---|---|---|
| Legende: -H Wasser (Referenz) -L Laccasebehandlung -D denaturierte Laccase | | | | |

Zur Denaturierung wurde Laccase in 50 ml demineralisiertes Wasser bei 90°C bis zur Trockenheit erhitzt. Nachfolgend wurde der verbleibende Feststoff in 50 ml Phosphat-Pufferraufgenommen und in die entsprechenden Versuche eingesetzt.

Im Ergebnis zeigt sich, dass mit der erfindungsgemäßen Behandlung der entsprechenden Oberflächen eine deutliche Verringerung des Wasserkontaktwinkels (Wasserrandwinkel) möglich ist.

## Patentansprüche

1. Verfahren zur Aktivierung einer Oberfläche durch Erhöhung der Hydrophilie oder für die Anbindung von Zielstrukturen, ausgewählt aus der Gruppe bestehend aus Proteinen, Zellen, Kohlenhydraten, Peptiden und Aminosäuren, umfassend den Schritt:
- Inkubation der Oberfläche mit einer Oxidoreduktase, ausgewählt aus der Gruppe E.C. 1.10 (Oxidoreduktasen, die an Diphenolen und ähnlichen Substanzen als Donoren agieren) nach der Klassifizierung nach dem IntEnz (Integrated relational Enzyme database des Swiss Institute of Bioinformatics (SIB)).

2. Verfahren nach Anspruch 1, wobei die Inkubation ohne die Zugabe einer Verbindung erfolgt, die eine di- oder tri-Hydroxyphenylgruppe umfasst.

3. Verfahren nach Anspruch 1 oder 2, wobei die Inkubation ohne die Zugabe Redoxmediators erfolgt.

4. Verfahren nach Anspruch 1, wobei die Inkubation unter Zugabe einer Verbindung erfolgt, die eine di- oder tri-Hydroxyphenylgruppe umfasst.

5. Verfahren nach Anspruch 4, wobei die Inkubation unter Zugabe einer Verbindung erfolgt, ausgewählt aus der Gruppe bestehend aus DOPA, Kaffeesäure und Dopamin.

6. Verfahren nach einem der vorangehenden Ansprüche, wobei das Material der Oberfläche ausgewählt ist aus der Gruppe bestehend aus Kunststoffen, Keramik, Glas, Kohlenhydraten und magnetischen Partikeln.

7. Verfahren nach Anspruch 6, wobei das Material der Oberfläche ausgewählt ist aus der Gruppe bestehend aus Polyolefinen, Polystyrol, Polypropylen, Polyethylen, Polycarbonat, Polyacryl, Polymethacryl, Cycloolefincopolymere und deren Derivaten sowie deren Mischungen.

8. Verfahren nach einem der vorangehenden Ansprüche wobei die Aktivierung der Oberfläche in Anwesenheit der Zielstruktur erfolgt.

9. Verfahren nach einem der vorangehenden Ansprüche, wobei die Inkubation der Oberfläche mit einer Lösung von Oxidoreduktase erfolgt und die Lösung bei Raumtemperatur bis zur vollständigen Verdunstung des Lösungsmittels stehen gelassen wird.

10. Verfahren nach einem der vorangehenden Ansprüche, wobei nach der Inkubation die Oxidoreduktase vollständig von der Oberfläche entfernt wird.

11. Verfahren nach einem der vorangehenden Ansprüche, wobei die Oxidoreduktase eine Laccase ist.

12. Verwendung einer Oxidoreduktase, ausgewählt aus der Gruppe E.C. 1.10 (Oxidoreduktasen, die an Diphenolen und ähnlichen Substanzen als Donoren agieren) zur Aktivierung einer Oberfläche durch Erhöhung der Hydrophilie oder zur Aktivierung einer Oberfläche für die Anbindung Zielstrukturen, ausgewählt aus der Gruppe bestehend aus Proteinen, Zellen Proteinen, Zellen, Kohlenhydraten, Peptiden und Aminosäuren.

13. Verwendung nach Anspruch 12, wobei keine Zugabe einer Verbindung erfolgt, die eine di- oder tri-Hydroxyphenylgruppe umfasst.

## Claims

1. A method for activating a surface by increasing the hydrophilicity or for binding target structures selected from the group consisting of proteins, cells, carbohydrates, peptides and amino acids, comprising the step of:
- incubation of the surface with an oxidoreductase, selected from the group E.C. 1.10 (oxidoreductases acting on diphenols and related substances as donors) in accordance with the classification according to IntEnz (Integrated relational Enzyme database of the Swiss Institute of Bioinformatics (SIB)).

2. The method according to claim 1, wherein incubation is carried out without the addition of a compound which comprises a di- or tri-hydroxyphenyl group.

3. The method according to claim 1 or 2, wherein incubation is carried out without the addition of redox mediator.

4. The method according to claim 1, wherein incubation is carried out with the addition of a compound which comprises a di- or tri-hydroxyphenyl group.

5. The method according to claim 4, wherein incubation is carried out with the addition of a compound selected from the group consisting of DOPA, caffeic acid and dopamine.

6. The method according to one of the preceding claims, wherein the material of the surface is selected from the group consisting of plastics, ceramics, glass, carbohydrates and magnetic particles.

7. The method according to claim 6, wherein the material of the surface is selected from the group consisting of polyolefins, polystyrene, polypropylene, polyethylene, polycarbonate, polyacrylic, polymethacrylic, cycloolefin copolymers and derivatives thereof and mixtures thereof.

8. The method according to one of the preceding claims, wherein the surface is activated in the presence of the target structure.

9. The method according to one of the preceding claims, wherein the surface is incubated with a solution of oxidoreductase and the solution is left to stand at room temperature until the solvent has completely evaporated.

10. The method according to one of the preceding claims, wherein after incubation the oxidoreductase is completely removed from the surface.

11. The method according to one of the preceding claims, wherein the oxidoreductase is a laccase.

12. Use of an oxidoreductase, selected from the group E.C. 1.10 (oxidoreductases acting on diphenols and related substances as donors), for activating a surface by increasing the hydrophilicity or for activating a surface for binding target structures, selected from the group consisting of proteins, cellular proteins, cells, carbohydrates, peptides and amino acids.

13. The use according to claim 12, wherein no compound comprising a di- or tri-hydroxyphenyl group is added.

## Revendications

1. Procédé pour l'activation d'une surface par augmentation de l'hydrophilie ou pour la liaison de structures cibles choisies dans le groupe consistant en protéines, cellules, glucides, peptides et acides aminés, comprenant l'étape suivants :
- l'incubation de la surface avec une oxydoréductase, choisie dans le groupe E.C. 1.10 (oxydoréductases qui agissent sur des diphénols et des substances similaires comme donneurs) d'après la classification selon l'IntEnz (Integrated relational Enzyme database de l'Institut suisse de bio-informatique (SIB)).

2. Procédé selon la revendication 1, dans lequel l'incubation s'effectue sans l'addition d'un composé qui comprend un groupe di- ou tri-hydroxyphényle.

3. Procédé selon la revendication 1 ou 2, dans lequel l'incubation s'effectue sans l'addition d'un médiateur redox.

4. Procédé selon la revendication 1, dans lequel l'incubation s'effectue en ajoutant un composé qui comprend un groupe di- ou tri-hydroxyphényle.

5. Procédé selon la revendication 4, dans lequel l'incubation s'effectue en ajoutant un composé choisi dans le groupe consistant en DOPA, acide caféique et dopamine.

6. Procédé selon l'une des revendications précédentes, dans lequel le matériau de la surface est choisi dans le groupe consistant en matières plastiques, céramique, verre, glucides et particules magnétiques.

7. Procédé selon la revendication 6, dans lequel le matériau de la surface est choisi dans le groupe consistant en polyoléfines, polystyrène, polypropylène polyéthylène, polycarbonate, polyacryle, polyméthacryle, copolymères de cyclo-oléfine et leurs dérivés ainsi que leurs mélanges.

8. Procédé selon l'une des revendications précédentes, dans lequel l'activation de la surface s'effectue en présence de la structure cible.

9. Procédé selon l'une des revendications précédentes, dans lequel l'incubation de la surface s'effectue avec une solution d'oxydoréductase et on laisse reposer la solution à température ambiante jusqu'à évaporation complète du solvant.

10. Procédé selon l'une des revendications précédentes, dans lequel après l'incubation, l'oxydoréductase est complètement enlevée de la surface.

11. Procédé selon l'une des revendications précédentes, dans lequel l'oxydoréductase est une laccase.

12. Utilisation d'une oxydoréductase choisie dans le groupe E.C. 1.10 (oxydoréductases qui agissent sur des diphénols et des substances similaires comme donneurs) pour l'activation d'une surface par augmentation de l'hydrophilie ou pour l'activation d'une surface pour la liaison de structures cibles, choisies dans le groupe consistant en protéines, protéines de cellules, cellules, glucides, peptides et acides aminés.

13. Utilisation selon la revendication 12, dans laquelle aucune addition d'un composé qui comprend un groupe di- ou tri-hydroxyphényle ne s'effectue.
